# EUROPEAN PATENT APPLICATION

(11) **EP 1 683 529 A1**
(43) Date of publication of application: **26.07.2006**
(21) Application number: 06001401.6
(22) Date of filing: 24.01.2006
(51) Int. Cl.: A61K 47/48, C07D 487/22, C07K 14/795, A61K 31/409

(54) **Inclusion compound of porphyrin metal complex and albumin**

(30) Priority: 25.01.2005 JP 2005016691
(71) Applicant: NIPRO CORPORATION, Kita-ku, Osaka-shi, Osaka-fu, 531-8510 (JP)
(72) Inventor: Kida, Yoshinori NIPRO CORPORATION, Osaka-shi Osaka-fu, 531-8510 (JP); Fukutomi, Ippei NIPRO CORPORATION, Osaka-shi Osaka-fu, 531-8510 (JP); Katayama, Naohisa NIPRO CORPORATION, Osaka-shi Osaka-fu, 531-8510 (JP); Kai, Toshiya NIPRO CORPORATION, Osaka-shi Osaka-fu, 531-8510 (JP)
(74) Representative: Albrecht, Thomas

(57) **Abstract**

A novel inclusion compound obtained by including in albumin a tetraphenylporphyrin metal complex represented by the following chemical formula (1) and having 2-position side chains: 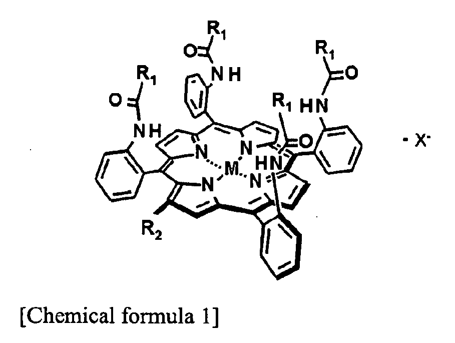 where R1 is a cyclohexyl group or the like; R2 represents a substituent; M represents a transition metal atom or metal ion belonging to Groups 6 to 10 of the periodic table; X⁻ represents a halogen ion; and the number of X⁻ is obtained by subtracting 2 from a valence of the metal ion.

## Description

### Technical Field of the Invention

The present invention relates to an oxygen carrier used in medical fields for oxygen supply to an ischemic site or tumor tissue, for blood transfusion to a patient with massive bleeding, for an organ preservation perfusion fluid, for an extracorporeal circulation fluid, and for a cell culture medium, and capable of reversibly adsorbing and desorbing oxygen.
More specifically, the present invention relates to a novel compound including in albumin a porphyrin metal complex as an oxygen adsorbing and desorbing site, and to an oxygen carrier containing the novel compound as an active component.

### Background of the Invention

In order to recover a patient from a hemorrhagic shock, a technique of replenishing a plasma expander having colloidal osmotic pressure substantially equal to that of blood in a living body has been heretofore employed. However, hemorrhage of 30% or more of a circulating blood volume causes insufficient oxygen supply to peripheral tissues and requires administration of the plasma expander and administration of an oxygen carrier.

Conventionally, natural blood containing natural red blood cells or a red blood cell concentrated liquid has been used as such oxygen carrier. However, in order to prevent blood clotting due to an antigen-antibody reaction, the blood types of a donor and a recipient must match and a cross matching must be performed for use. The natural blood or the red blood cell concentrated liquid has a short effective storage life of 3 weeks (4EC), and frozen blood which can be stored for a long period of time by frozen storage has problems such as high cost and easily-caused hemolysis due to osmotic shock in use. Further, onset of infectious diseases such as hepatitis and AIDS is another problem of concern.

As an oxygen carrier for solving the above-mentioned problems, a porphyrin iron (II) complex present in hemoglobin or myoglobin has attracted attention because the complex is capable of reversibly adsorbing and desorbing oxygen molecules. Many researches on an oxygen carrier employing a synthetic complex similar to such a natural porphyrin iron (II) complex have been heretofore reported (see Non-patent Documents 1 and 2, for example, identified below).

Meanwhile, in the case where a synthetic compound (in particular, a synthetic porphyrin metal complex or the like) is used for medical applications such as artificial red blood cells, an organ preservation liquid, or an oxygen supply liquid for an artificial lung or the like by reproducing its functions under physiological conditions (in physiological saline (pH 7.4), at room temperature or 37EC), the following conditions must be satisfied.
(i) In general, an axial base ligand must be present for increasing an oxygen-binding affinity. However, an imidazole derivative widely used as such a ligand may provide a pharmacological effect and may exhibit high toxicity in a living body. Thus, the concentration of the imidazole derivative must be suppressed to minimum.
(ii) The porphyrin metal complex must be not only solubilized in water but also embedded and fixed in a microscopic hydrophobic environment, to thereby prevent oxidation of a central metal by protons and oxidation through a µ-oxo dimer and maintain a stable oxygen-coordinated complex.

Regarding the condition (i), there is already proposed a method involving synthesizing a porphyrin compound having an imidazolyl group bound to a porphyrin ring through a covalent bond, that is, a substituted porphyrin compound having an axial base in a molecule (a molar ratio of porphyrin / imidazole suppressed to 1 : 1 as a minimum necessary ratio) to form a stable oxygen-coordinated complex (see Patent Document 1, identified below).
A porphyrin complex synthesized by binding an axial base through an ester bond, in particular, has high biodegradability and is highly advantageous for administration into a living body. This porphyrin complex is known to be useful as a highly safe oxygen binding site (porphyrin metal complex).

Regarding the condition (ii), a micelle of a surfactant or a bilayer vesicle of a phospholipid is known to be used. However, the micelle has a dynamic form, is less stable compared with that of the bilayer vesicle, and forms a less hydrophobic environment. Thus, the bilayer vesicle having a relatively stable form and capable of providing a sufficiently hydrophobic region is used more often as a carrier for providing the complex with a hydrophobic environment. Development of an oxygen carrier capable of carrying oxygen stably even under physiological conditions by dispersing with high orientation a porphyrin metal complex within a bilayer of a phospholipid vesicle has continuously advanced.

From the viewpoints of introducing a terminal hydrophilic alkyl substituent into a porphyrin ring and providing an amphiphilic structure to porphyrin for embedding the porphyrin with high orientation into a hydrophobic environment of the phospholipid bilayer, various amphiphilic porphyrin iron complexes have already been synthesized. These porphyrin complexes are each embedded and orientated in the phospholipid bilayer, to thereby develop a series of oxygen carriers effective in an aqueous phase (see Patent Document 2, for example, identified below).
However, these oxygen carriers each use a large amount of the phospholipid, and have problems in production on an industrial scale and biocompatibility such as metabolism.

Meanwhile, as an oxygen carrier using no phospholipid, an inclusion compound of albumin and heme (albumin-heme) has been studied. An example of albumin-heme is a compound obtained by including into a hydrophobic pocket of albumin a 2-[8- (2-methyl-1-imidazolyl)] octanoyloxymethyl]-5,10,15,20-tetrakis (α,α,α,α-o-pivalioylamino) phenylporphinatoiron (II) or the like, which is a tetraphenylporphyrin iron derivative (see Non-patent Document 3, identified below).

However, these oxygen carriers still have room for improvement in biocompatibility such as metabolism and production on an industrial scale.

[Patent Document 1] JP 06-271577 A
[Patent Document 2] JP 58-213777 A
[Non-patent Document 1] J. P. Collman, Accounts of Chemical Research, 10, p. 265 (1977)
[Non-patent Document 2] F. Basolo, B. M. Hoffman, J. A. Ibers, Accounts of Chemical Research, 8, p. 384 (1975)
[Non-patent Document 3] E. Tsuchida et al., Bioconjugate Chemistry, vol. 8, p. 534-538, 1997

### [Disclosure of the Invention]

### [Problem to be solved by the Invention]

An object of the present invention is to provide: a novel compound which has excellent biocompatibility as an oxygen carrier containing a porphyrin metal complex as an oxygen adsorbing and desorbing site, and which can be produced on an industrial scale with relative ease; and a method of producing the novel compound.

### Summary of the Invention

In view of attaining the above-mentioned object, the inventors of the present invention have conducted extensive studies on molecular design and function development of an oxygen carrier (porphyrin metal complex) capable of carrying oxygen stably under physiological conditions, and on a carrier having high biocompatibility and capable of providing the porphyrin metal complex with a hydrophobic environment.
As a result, the inventors of the present invention have produced a compound including a tetraphenylporphyrin metal complex represented by the following chemical formula (1) and having 2-position side chains, and albumin accounting for 50 to 55% of plasma proteins and capable of carrying various compounds in a living body, and have found that this compound is useful as an oxygen carrier having excellent biocompatibility.

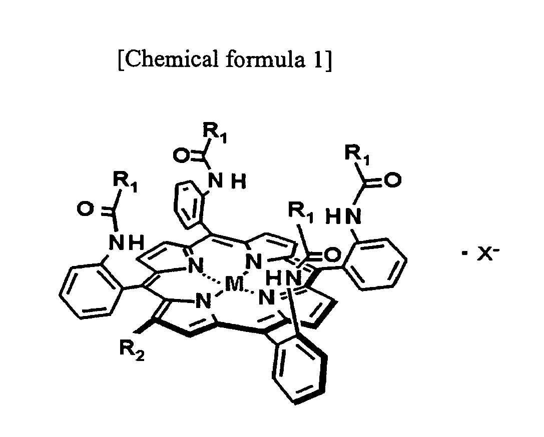

(in chemical formula (1): R1 is a cyclohexyl group or the like; R2 represents a substituent; M represents a transition metal atom or metal ion belonging to Groups 6 to 10 of the periodic table; X⁻ represents a halogen ion; and the number of X⁻ is obtained by subtracting 2 from a valence of the metal ion).

That is, the present invention relates to:
(1): A novel inclusion compound obtained by including in albumin a tetraphenylporphyrin metal complex represented by chemical formula (1) and having 2-position side chains;
(2): The inclusion compound according to the above item (1), in which the tetraphenylporphyrin metal complex is represented by the following chemical formula (2):

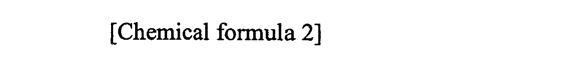

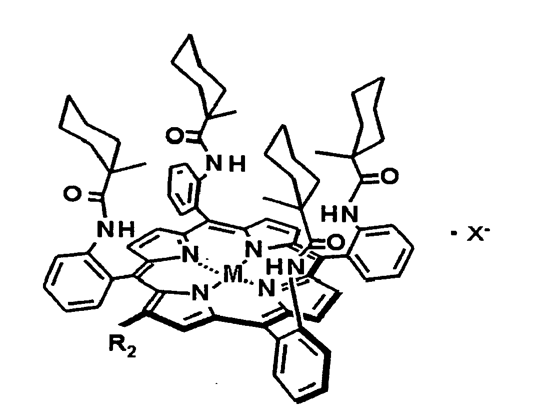

(in chemical formula (2): R2 represents a substituent; M represents a transition metal atom or metal ion belonging to Groups 6 to 10 of the periodic table; X⁻ represents a halogen ion; and the number of X⁻ is obtained by subtracting 2 from a valence of the metal ion);
(3): The inclusion compound according to the above item (1), in which R2 is represented by the following chemical formula (3):

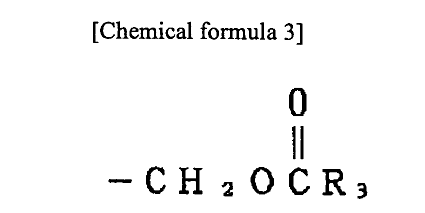

(in chemical formula (3), R3 represents an alkyl group having 1 to 18 carbon atoms or a group represented by the following chemical formula (4):)

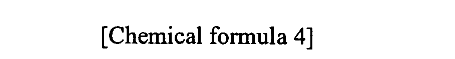

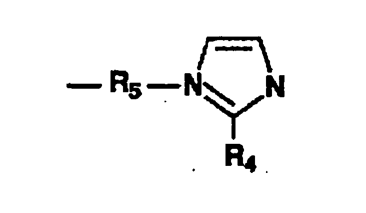

(in chemical formula (4): R4 represents a group which does not inhibit coordination to the central metal M of imidazole bound to R4; and R5 represents an alkylene group);
(4): The inclusion compound according to the above item (1), in which M represents iron, cobalt, or chromium;
(5): The inclusion compound according to the above item (1), in which M represents divalent iron, trivalent iron, or divalent cobalt;
(6): The inclusion compound according to the above item (3), in which R4 represents hydrogen, a methyl group, an ethyl group, or a propyl group;
(7): The inclusion compound according to the above item (3), in which R5 represents an alkylene group having 1 to 10 carbon atoms;
(8): The inclusion compound according to the above item (1), in which albumin comprises human serum albumin or recombinant human serum albumin;
(9): An oxygen carrier comprising the inclusion compound according to the above item (1) as an active component; and
(10): A pharmaceutical composition comprising the oxygen carrier according to the above item (9).
Brief Description of the Drawings
[Fig. 1] A diagram showing a change in weight after blood exchange in Experiment 1.
[Fig. 2] Diagrams showing biochemical data after blood exchange in Experiment 1.

### Detailed Description of the Invention

The porphyrin metal complex used in the present invention is represented by chemical formula (1) and is a tetraphenylporphyrin metal complex having 2-position side chains in which a central metal is coordinated to a porphyrin ring derivative. The porphyrin ring includes a large cyclic compound in which four pyrrole rings are alternately bound to four methine groups at α-positions, and derivatives thereof.
The porphyrin metal complex of the present invention has such a feature that, in chemical formula (1), R1 is a cyclohexyl group or the like.

In chemical formula (1), a substituent (R2) at the 2-position is represented by the following chemical formula (3), for example.

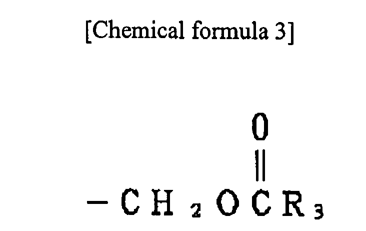

(in chemical formula (3), R3 represents an alkyl group having 1 to 18 carbon atoms or a group represented by the following chemical formula (4):)

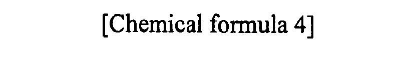

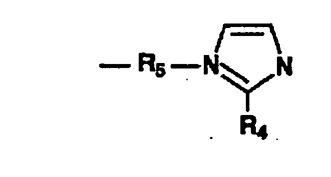

(in chemical formula (4): R4 represents a group which does not inhibit coordination of imidazole bound to R4 to a central metal; and R5 represents an alkylene group)

In chemical formula (4), R4 preferably represents hydrogen or a lower alkyl group such as a methyl group, an ethyl group, or a propyl group, and R5 preferably represents an alkylene group having 1 to 10 carbon atoms.

Of the tetraphenylporphyrin metal complexes of the present invention represented by chemical formula (1), a specific example of a complex capable of binding oxygen is a complex having a divalent central metal (M) and one base coordinated. Such a complex is represented by the following chemical formula (5) or chemical formula (6).

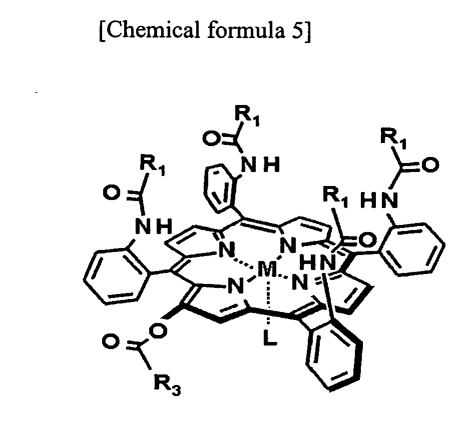

In chemical formula (5), L represents a nitrogen-based axial ligand such as an imidazole derivative.

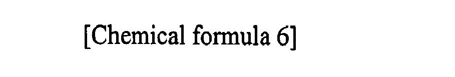

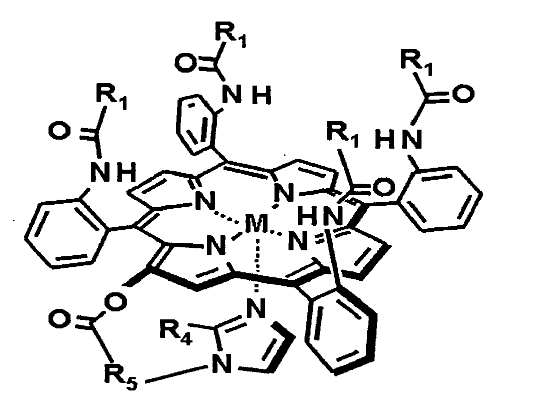

Of the tetraphenylporphyrin metal complexes of the present invention represented by chemical formula (1), an example of a complex serving as an effective oxygen adsorbing and desorbing agent or oxygen carrier is a complex having a divalent central metal (M). In the case where the substituent at a 2-position itself is an imidazole derivative serving as an axial base, imidazole in a molecule may be coordinated to the porphyrin central metal (M). Thus, the imidazole derivative itself may provide oxygen-binding affinity without external addition of an excess amount of the axial base. That is, a porphyrin compound having an axial base bound thereto in a molecule requires no addition of a large amount of the imidazole derivative as an axial base. In consideration of administration into a living body, the porphyrin compound may serve as a useful material having a significantly reduced concentration of the axial base.

The central metal (M in the general formula (1)) in the porphyrin metal complex of the present invention is a transition metal atom belonging to Groups 6 to 10 of the periodic table, and may be in the form of a metal ion. Preferred examples thereof include iron, cobalt, and chromium, and more preferred examples thereof include divalent iron (iron (II)), trivalent iron (iron (III)), and divalent cobalt (cobalt (II)). In the case where the central metal is iron, divalent iron has oxygen adsorbing and desorbing capacity.
The porphyrin metal complex of the present invention in the form of an iron (III) complex may be provided with oxygen-binding affinity by using an appropriate reducing agent (such as sodium dithionite or ascorbic acid) and reducing the central iron from trivalent to divalent through a conventional method.

A particularly preferred example of the porphyrin metal complex of the present invention is a tetraphenylporphyrin metal complex represented by the following chemical formula (7) and having a transition metal atom coordinated to a porphyrin ring.
In chemical formula (7), M represents a transition metal atom or metal ion belonging to Groups 6 to 10 of the periodic table.

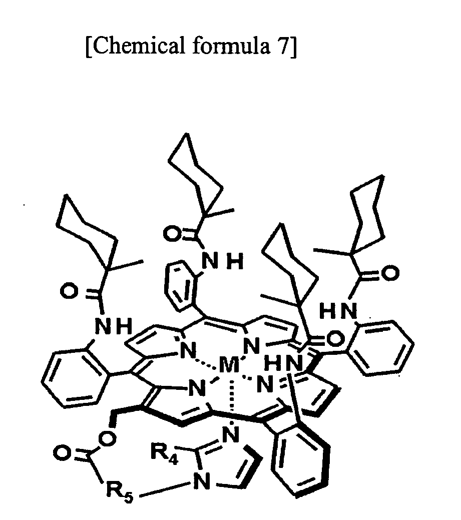

(In chemical formula (7): M represents a transition metal atom or metal ion belonging to Groups 6 to 10 of the periodic table; R4 represents a group which does not inhibit coordination of imidazole bound to R4 to a central metal; and R5 represents an alkylene group).

Of the porphyrin metal complexes, a particularly preferred example thereof is a complex represented by the following chemical formula (8) and having iron coordinated to a center of 2-[8-(2-methyl-1-imidazolyl) octanoyloxymethyl] - 5,10,15,20 - tetrakis [α,α,α,α- o - (1- methylcyclohexanoylamino) phenylporphyrin].

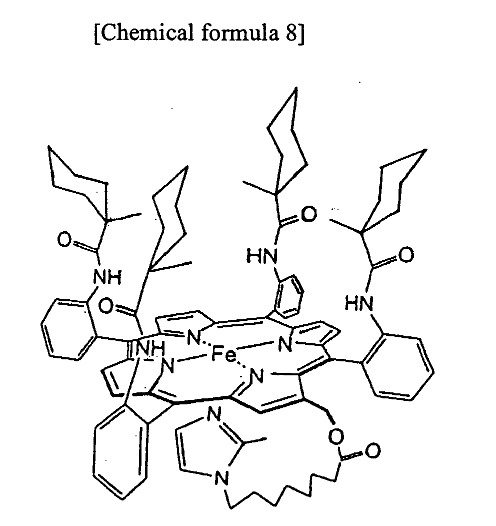

In the porphyrin metal complex represented by chemical formula (8), it is important that an alkylimidazolyl group be bound to a 2-position of the porphyrin ring. Based on the studies of the inventors of the present invention, a porphyrin complex having no such imidazolyl group in a molecule degrades immediately without forming a stable oxygen carrier in an aqueous phase even if a slightly excess amount of an imidazole derivative (such as 1-methylimidazole) is added externally.

The inclusion compound of a porphyrin metal complex and albumin of the present invention is obtained by embedding and fixing (including) a porphyrin metal complex in a hydrophobic region formed inside albumin. The number of porphyrin metal complexes bound to 1 mole of albumin (such as human serum albumin) is generally about 1 to 8. The number of porphyrin metal complexes included or bound to 1 mole of albumin can be determined by producing a Scatchard plot (C. J. Halfman, T. Nishida, Biochemistry, 11, p. 3493 (1972)), for example.

Examples of albumin to be used in the present invention include human serum albumin, recombinant human serum albumin, and bovine serum albumin, and the origin of albumin is not particularly limited. In view of application to humans, human serum albumin or human serum albumin produced through a gene recombination technique (hereinafter, referred to as recombinant human serum albumin) is preferred, and recombinant human serum albumin is particularly preferred.

Recently, high purity recombinant human serum albumin having completely identical structure, composition, and physicochemical properties to those of human serum albumin has been developed owing to the development of the gene recombination technique (see Yokoyama, Omura, Rinsho Bunshi Igaku, p. 1939, (1993)). A porphyrin metal complex-recombinant human serum albumin inclusion compound obtained by including a substituted porphyrin metal complex into recombinant human serum albumin can be provided as a totally synthetic system, and thus can be produced on an industrial scale with relative ease.
Further, albumin is a plasma protein, and is more advantageous for application to a living body, in particular, for use as a red blood cell alternative as compared with a system employing a phospholipid vesicle.

Albumin is a simple protein with a main function of adjusting a colloid osmotic pressure in blood, but also serves as a transport protein for nutritive substances, metabolites, chemicals, and the like. The present invention has been completed focusing on the non-specific binding capacity of albumin, which is not observed in other proteins.

The oxygen carrier of the present invention is a compound or structure containing the inclusion compound obtained by including in albumin the porphyrin metal complex, and is used as a red blood cell alternative in blood transfusion, an issue injury preventing agent for an ischemic site, a therapeutic agent for an ischemic disease such as cardiac infarction, or a sensitizer for radiotherapy of a tumor.
Further, the scope of the present invention includes various pharmaceutical compositions each containing such an oxygen carrier and having a stabilizer or the like added thereto.

The tetraphenylporphyrin metal complex of the present invention represented by the general formula (1) can be produced through various known production methods. For example, 2-[8-(2-methyl-1-imidazolyl)octanoyloxymethyl]-5,10,15,20-tetrakis[α,α,α,α-o-(1-methylcyclohexanoylamino)phenyl] porphinatoiron(II) can be produced through a production method described in T. Komatsu et al., Bioconjugate Chemistry 13, p.397-402,2002.

Next, the inclusion compound of a porphyrin metal complex and albumin of the present invention can be prepared as described below. First, a porphyrin metal complex is dissolved in a solvent (such as ethanol or methanol), and an aqueous solution (examples of a solvent include water, phosphate buffer (pH 5 to 9), physiological saline, Krebs-Ringer solution) of albumin (such as human serum albumin) is added thereto. Then, the whole is shaken lightly. The obtained aqueous dispersion is concentrated to about 10% of a total volume through ultrafiltration (by using an ultrafiltration membrane of an ultrafiltration molecular weight of 20,000 to 40,000, for example). Water or the like is added thereto again, and the ultrafiltration operation is repeated, to thereby obtain a porphyrin metal complex-albumin inclusion compound. No precipitation, aggregation, or the like is observed in the dispersion stored at 4 to 35EC for several months, and the dispersion is stable.

In the case where the central metal of the porphyrin metal complex is iron (III), the central iron is desirably reduced from trivalent to divalent through a conventional method such as adding a reducing agent (such as an aqueous solution of sodium dithionite, ascorbic acid, or the like), to thereby impart oxygen-binding affinity.
The reduction can be performed not only by addition of a reducing agent, but also by adding palladium carbon/hydrogen gas. For example, the porphyrin iron (III) complex is dissolved in dry dichloromethane, benzene, toluene, or the like, and a small amount of palladium carbon is added thereto. Then, a hydrogen gas may be sufficiently blown into the mixture at room temperature, to thereby reduce the central iron. After the reduction, palladium carbon is separated through filtration, and a supernatant may be dried under vacuum for use.
The reduction is preferably performed before an inclusion reaction.

Hereinafter, the present invention will be described in more detail by using examples, but the present invention is not limited thereto.

### [Example 1]

(Production of porphyrin metal complex)
2-[8-(2-methyl-1-imidazolyl) octanoyloxymethyl]-5,10,15,20-tetrakis [α,α,α,α-o-(1-methylcyclohexanoylamino)phenyl]porphinatoiron(II) was produced through a production method described in T. Komatsu et al., Bioconjugate Chemistry 13, p.397-402,2002.

(Inclusion of porphyrin metal complex and albumin)
1.5 L of an aqueous 0.6 M L-ascorbic acid solution was added to an ethanol solution (1.6 L) of 2-[8-(2-methyl-1-imidazolyl) octanoyloxymethyl] -5,10,15,20-tetrakis [α,α,α,α-o-(1-methylcyclohexanoylamino)phenyl] porphinatoiron(II) (1.07 mM) in a carbon monoxide atmosphere, in order to reduce the porphinatoiron(II). Then the mixture was added to 6.5 L of an aqueous phosphate buffer solution (pH 7.4, 1/30 mM) of human serum albumin (0.27 mM), and the whole was stirred. While 60 L of an aqueous phosphate buffer solution (pH 7.4, 1/30 mM) was added to the obtained mixed liquid, a fixed ultrafiltration dialysis was performed by using an ultrafiltration device (ultrafiltration membrane, manufactured by Millipore Corporation, ultrafiltration molecular weight of 30,000), to thereby remove ethanol in the mixed liquid. The mixed liquid was concentrated to 300 mL, to thereby obtain a desired dispersion of a porphyrin metal complex-albumin inclusion compound (hereinafter, abbreviated as rHSA-FecycP): No precipitation, aggregation, or the like was observed in rHSA-FecycP (CO combined) stored at room temperature or 4EC for several months, and rHSA-FecycP was very stable.

rHSA-FecycP (CO combined) was diluted to 1/50, and the diluted solution was subjected to UV-vis absorption spectroscopy, exhibiting a maximum absorbance at a wavelength of 427 nm.

A UV-vis spectrum of rHSA-FecycP (CO combined) under photoirradiation (500 w) in a stream of an oxygen gas changed immediately, exhibiting a maximum absorbance at a wavelength of 424 nm. The result indicated that a porphyrin iron (II) complex included in albumin was converted into an oxygenated complex. The LTV-vis spectrum of rHSA-FecycP (O₂ combined) in a stream of nitrogen changed immediately, exhibiting a maximum absorbance at a wavelength of 443 nm. The result indicated that oxygen was removed from the porphyrin iron (II) complex included in albumin. The UV-vis spectrum of rHSA-FecycP (Deoxy body) in a stream of oxygen changed immediately to a spectrum of an oxygenated complex. The result confirmed that adsorption and desorption of oxygen was reversible. Alternating of streams of oxygen and nitrogen was repeated, to thereby allow repeated adsorption and desorption of oxygen.

rHSA-FecycP had an oxygen affinity (P_{1/2} (O₂)) of 35 Torr (37EC). The oxygen affinity is the pressure necessary to maintain the condition that half of the number of porphyrin metal complexes are combined with oxygen. An oxygen carrying efficiency between a lung (110 Torr) and a peripheral tissue (40 Torr) estimated from an oxygen binding and dissociation equilibrium curve was about 20% (37EC), indicating that the inclusion compound of the present invention acts efficiently as an oxygen carrier for a red blood cell alternative. An enthalpy/entropy change in oxygen binding revealed that the behavior of an oxygen-coordinated complex in the inclusion compound of the present invention is substantially identical to that of hemoglobin in red blood cells.

(Experiment 1)
A rat blood exchange model was used for rHSA-FecycP obtained in Example 1, and safety of rHSA-FecycP in vivo was evaluated by weight change and blood biochemical values as indices.

(Test substance and control substance)
rHSA-FecycP obtained in Example 1 was used as a test substance, and recombinant human serum albumin (25% preparation, available from BIPHA CORPORATION, hereinafter, referred to as rHSA) was used as a control substance. The test substance was oxygenated, stored at 4EC, and used within 8 hours after the oxygenation. 25% rHSA was diluted five times with physiological saline (Otsuka normal saline, available from Otsuka Pharmaceutical Factory, Inc.) and prepared into an administration liquid of concentration of 5% in use.

(Used chemicals and reagents)
The chemicals and reagents used except the test substance and the control substance were as follows: diethyl ether (available from Wako Pure Chemical Industries, Ltd.); sevoflurane (trade name, SEVOFRANE, available from Maruishi Pharmaceutical Co., Ltd.); heparin (trade name, Novo heparin injection 1000, available from Aventis Pharma Japan); and physiological saline (trade name, Otsuka normal saline, available from Otsuka Pharmaceutical Factory, Inc.).

(Used animals)
Crj: Wistar male rats (7 week old) were purchased from Charles River Japan, Inc., and 36 well-grown rats were subjected to a blood exchange test. The rats were classified into three groups of a control group (no blood exchange performed), an rHSA group (blood exchange performed by using rHSA), and a cyc group (blood exchange performed by using rHSA-FecycP) with 12 rats for each group. Note that blood was collected from 6 live rats of the 12 rats in each group a day after the blood exchange, and blood was collected from the remaining 6 live rats 7 days after the blood exchange.

(Treatment)
The rats were each preliminarily anesthetized by using ether, then anesthetized by using sevoflurane (trade name, SEVOFRANE, available from Maruishi Pharmaceutical Co., Ltd., 2.0% inhalation), and fixed in a dorsal position for shaving hair of a treatment site by using an animal clipper. A right femoral region was dissected, and a tip of a catheter filled with 50 U/ml of heparin was inserted into a right femoral vein, to thereby provide a blood removal route. The rats of the rHSA group and cyc group were subjected to 20% blood exchange. That is, 1 ml of blood was collected from the right femoral vein of each rat, and then 1 ml of the test substance or control substance was administered from the right femoral vein as one operation. The operation was repeated four times in total. Blood was collected from an abdominal vena cava 1 day and 7 days after the completion of the blood exchange.

(Observation of general symptoms)
General symptoms were observed before the blood exchange, and 1 day, 3 days, and 7 days after the completion of the blood exchange. No change in general symptoms was observed for all three groups.

(Change in weight)
Weight of each rat was measured before the blood exchange, and 1 day, 3 days, and 7 days after the completion of the blood exchange. Note that weight before the blood exchange was subtracted from the weight 1 day, 3 days, or 7 days after the blood exchange, to thereby calculate a change in weight (g). Fig. 1 shows a change in weight of the rats of each group until 7 days after the blood exchange. The observation was continued until 7 days after the completion of the blood exchange, but no significant difference was observed among the control group, the rHSA group, and the cyc group.

(Biochemical test of blood)
AST, ALT, LDH, LAP, CRN, and BUN in plasma were quantitatively determined. Fig. 2 shows the results of quantitative determination of plasma of the rats of each group 1 day and 7 days after the completion of the blood exchange. The collected venous blood was subjected to centrifugation to collect plasma, and immediately subjected to frozen storage (set temperature of -20EC).

(1 day after completion of blood exchange)
No significant difference was observed in amounts of AST, ALT, LDH, LAP, CRN, and BUN in plasma for all three groups.

(7 days after completion of blood exchange)
No significant difference was observed in amounts of AST, ALT, LDH, LAP, CRN, and BUN in plasma for all three groups.

Experiment 1 and 2 reveal that rHSA-FecycP obtained in Example 1 exhibits excellent oxygen adsorbing and desorbing capacity even under physiological conditions and has excellent biocompatibility.

The inclusion compound of the present invention can be used as an oxygen carrier for various medical applications, and can be used as a red blood cell alternative in blood transfusion, an issue injury preventing agent of an ischemic site, a therapeutic agent for an ischemic disease such as cardiac infarction, or a sensitizer for radiotherapy of a tumor.

### Effect of the Invention

The oxygen carrier containing the inclusion compound of a porphyrin metal complex and albumin (hereinafter, referred to as a porphyrin-albumin inclusion compound) of the present invention has excellent biocompatibility and exhibits excellent oxygen adsorbing and desorbing capacity even under physiological conditions.
Further, the porphyrin-albumin inclusion compound of the present invention and an oxygen carrier containing the porphyrin-albumin inclusion compound as an active component can be produced on an industrial scale with relative ease.

In the porphyrin-albumin inclusion compound of the present invention, the porphyrin metal complex which is an oxygen adsorbing site is fixed inside a hydrophobic environment of albumin, to thereby completely suppress oxidation of a central metal by protons and an oxidation degradation process through a µ-oxo dimer. As a result, the inclusion compound of the present invention can maintain a stable oxygen-coordinated complex even in an aqueous phase.

Further, the porphyrin-albumin inclusion compound of the present invention forms a stable oxygen-coordinated complex immediately after the compound is brought into contact with oxygen. The oxygen-coordinated complex is capable of adsorbing and desorbing oxygen in accordance with an oxygen partial pressure. The adsorption and desorption of oxygen may be repeated stably and reversibly in accordance with a difference of the oxygen partial pressure. Meanwhile, binding and dissociation of oxygen are quick, and the inclusion compound of the present invention may serve as a semiartificial oxygen carrier capable of carrying oxygen efficiently even in blood flow of a living body. Alternatively, an inclusion compound obtained by using recombinant human serum albumin may server as a totally synthetic oxygen carrier.

The porphyrin-albumin inclusion compound of the present invention can be produced easily. In particular, an inclusion compound obtained by using recombinant human serum albumin or the like is suitable for production on an industrial scale.

## Claims

1. An inclusion compound obtained by including in albumin a tetraphenylporphyrin metal complex represented by formula (1) and having 2-position side chains: 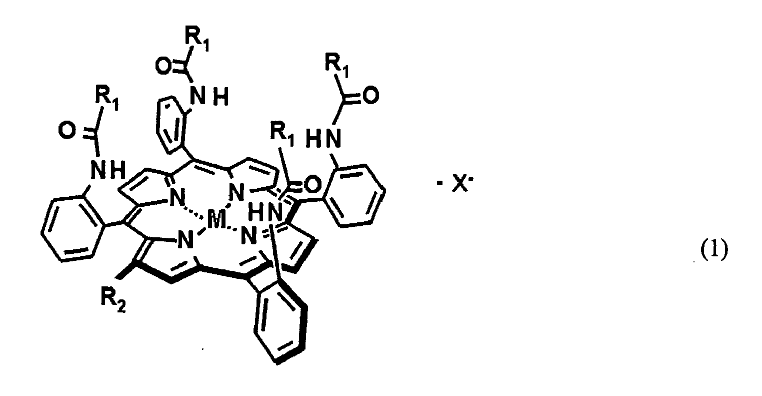 where R1 is a cyclohexyl group or the like; R2 is a substituent represented by chemical formula (3) or chemical formula (4): 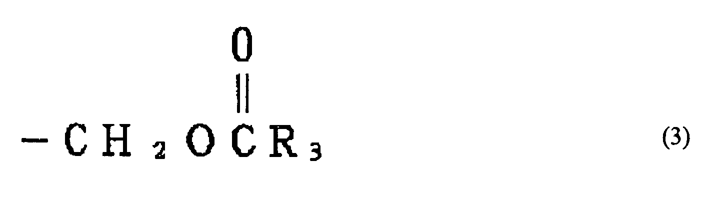 where R3 represents an alkyl group having 1 to 18 carbon atoms or a group represented by the following formula (4): 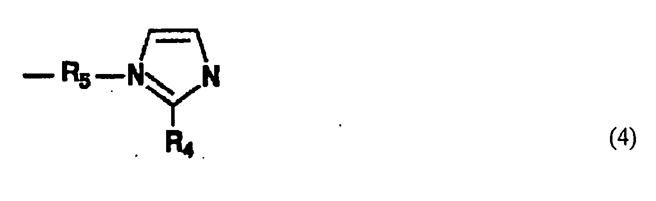 where R4 represents a group which does not inhibit coordination to M of imidazole bound to R4; and R5 represents an alkylene group;
M represents a transition metal atom or metal ion belonging to Groups 6 to 10 of the periodic table; X⁻ represents a halogen ion; and the number of X⁻ is obtained by subtracting 2 from a valence of the metal ion.

2. The inclusion compound according to claim 1, wherein the tetraphenylporphyrin metal complex is represented by the following formula (2): 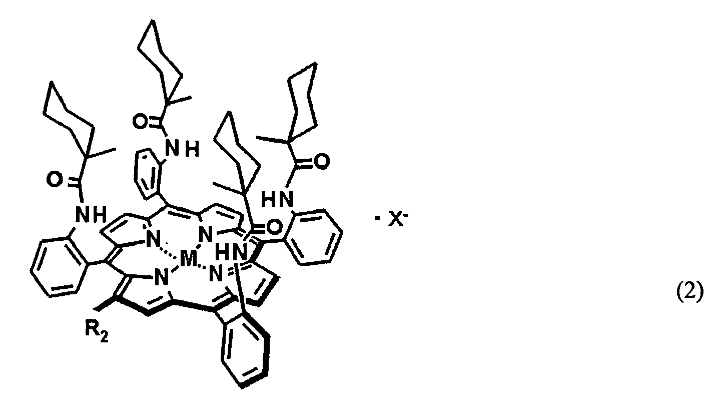 where R2 is a substituent as defined in claim 1; M represents a transition metal atom or metal ion belonging to Groups 6 to 10 of the periodic table; X⁻ represents a halogen ion; and the number of X⁻ is obtained by subtracting 2 from a valence of the metal ion.

3. The inclusion compound according to claim 1, wherein M represents iron, cobalt, or chromium.

4. The inclusion compound according to claim 1, wherein M represents divalent iron, trivalent iron, or divalent cobalt.

5. The inclusion compound according to claim 1, wherein R4 represents hydrogen, a methyl group, an ethyl group, or a propyl group.

6. The inclusion compound according to claim 1, wherein R5 represents an alkylene group having 1 to 10 carbon atoms.

7. The inclusion compound according to claim 1, wherein the albumin comprises human serum albumin or recombinant human serum albumin.

8. An oxygen carrier comprising the inclusion compound according to claim 1 as an active component.

9. A pharmaceutical composition comprising the oxygen carrier according to claim 8.
